# EUROPEAN PATENT APPLICATION

(11) **EP 1 609 859 A1**
(43) Date of publication of application: **28.12.2005**
(21) Application number: 04719080.6
(22) Date of filing: 10.03.2004
(51) Int. Cl.: C12N 15/09, C12N 15/82, C12N 5/14, A01H 5/00

(54) **TECHNIQUE OF REMOVING MARKER GENE BY TRANSIENT EXPRESSION OF SITE-SPECIFIC RECOMBINASE GENE**

(30) Priority: 12.03.2003 JP 2003067173
(71) Applicant: NATIONAL INSTITUTE OF AGROBIOLOGICAL SCIENCES, Tsukuba-shi, Ibaraki 305-8602 (JP)
(72) Inventor: TOKI, Seiichi, Nat. Inst. of Agrobiological Sci., Tsukuba-shi, Ibaraki 305-8602 (JP); ICHIKAWA, Hiroaki, Nat. Inst. of Agrobiological Sc, Tsukuba-shi, Ibaraki 305-8602 (JP); OSAKABE, Keishi, Tsukuba-shi, Ibaraki 3050035 (JP)
(74) Representative: Bösl, Raphael Konrad
(86) International application number: PCT/JP2004/003069
(87) International publication number: WO 2004/081211

(57) **Abstract**

The present inventors conceived that a marker gene may be efficiently and easily removed by using the floral dip method to transiently express high levels of a site-specific recombinase gene. It is considered that the present methods can also be applied to methods for transposing a transposon that does not have a transposase from a transformed plant that comprises the transposon having no transposase.

## Description

### Technical Field

The present invention relates to methods for efficiently and easily removing a DNA flanked by recognition sequences for a site-specific recombinase from a transformed plant comprising the DNA. Furthermore, the present invention relates to methods for efficiently and easily transposing a transposon that does not have a transposase from a transformed plant comprising the transposon having no transposase.

### Background Art

Methods that have been devised for removing a marker gene inserted in a genomic DNA involve: placing site-specific recombinase recognition sequences on both sides of the marker gene, and excising and removing the marker gene with a site-specific recombinase.
Specifically, one method excises and removes a marker gene by crossing an organism comprising the marker gene to which site-specific recombinase recognition sequences have been added, with an organism to which a site-specific recombinase gene has been introduced (Non-Patent Document 1). A second method transforms an organism with a marker gene and a site-specific recombinase protein gene controlled by an inducible promoter, both of which are placed in advance between the site-specific recombinase recognition sequences, and induces gene expression of the site-specific recombinase to excise and remove the marker gene, as needed (Non-Patent Document 2). However, these two methods are problematic in that the efficiency of excision and removal is extremely low due to insufficient expression levels of the site-specific recombinase. Furthermore, at present it is difficult to completely suppress the expression of a site-specific recombinase by the method described in Non-Patent Document 2. Thus, the high probability of losing the marker gene to the site-specific recombinase's enzymatic action is problematic when the marker gene is used to select transformants after a transformation treatment.

However, methods that can efficiently and easily remove a marker gene by transiently expressing high levels of a site-specific recombinase gene using an in planta transformation method are yet unknown.

Information on prior art relevant to the invention of the present application is shown below.
[Non-Patent Document 1] Onouchi H. *et al*. Visualization of site-specific recombination catalyzed by a recombinase from *Zygosaccaromyces rouxii* in *Arabidopsis thaliana*. (1995) Mol. Gen. Genet. 247,653-660.
[Non-Patent Document 2] Sugita K. *et al*. A transformation vector for production of marker-free transgenic plants containing a single copy transgene at high frequency. Plant J. (2000) 22, 461-469.

### Disclosure of the Invention

The present invention was made in view of the above circumstances. An objective is to provide methods for efficiently and easily removing a DNA flanked by site-specific recombinase recognition sequences from a transformed plant comprising the DNA. Another objective of the present invention is to provide methods for efficiently and easily transposing a transposon that does not have a transposase, from a transformed plant comprising the transposon having no transposase.

Upon close scrutiny of the Ye *et al*. paper "Guang-Ning Ye *et al*. (1999) Plant J., 19(3):249-257)", the present inventors noticed that when a GUS gene was introduced by the vacuum infiltration method (a type of in planta transformation method), an extremely strong transient expression of the GUS gene was observed in *Arabidopsis* plants. At the same time, Hodges's group reported that by transiently expressing an FLP gene in protoplasts derived from transformed maize, sequences flanked by the FLP-specific recognition sequence FRT could be excised (Lyznik L.A. *et al*. (1993) Nucleic Acids Res. 25, 21(4):969-975).

The present inventors then thought that it might be possible to remove marker genes easily and efficiently by transiently expressing high levels of a site-specific recombinase gene utilizing the floral dip method, a type of in planta transformation method that can induce strong transient expression of a foreign gene in the early stages of embryonic development.

First, a construct was generated using an FLP recombinase derived from budding yeast as the site-specific recombination protein, in which the FRT sequence, a recognition sequence for the FLP recombinase, was placed on both sides outside of the hygromycin-resistant gene cassette. Additionally, a mannopine synthase gene promoter and a bialaphos resistance gene were placed outside of the FRT sequences in this construct. In principle, if the region between FRT sequences is removed by FLP, the mannopine synthase gene promoter and the bialaphos resistance gene will become closer in position, thus conferring bialaphos resistance. Next, the present inventors used a pKO101-harboring *A. tumefaciens* EHA105 strain to generate transformed *Arabidopsis* plants by the floral dip method. Whether or not the hygromycin resistance gene is efficiently removed can be examined using bialaphos resistance as an index, and applying the floral dip method to the pKO101-introduced *Arabidopsis* plants using an FLP-harboring *A. tumefaciens* EHA105 strain, wherein the FLP functions under an early embryo-specific promoter.

This method is also considered applicable to methods for transposing a transposon that does not have a transposase from a transformed plant comprising the transposon having no transposase.

Specifically, the present invention provides the following (1) to (6).
(1) A method for removing a DNA flanked by site-specific recombinase recognition sequences, wherein the method comprises the steps of: using *Agrobacterium* to introduce a DNA encoding a site-specific recombinase into a transformed plant that comprises the DNA flanked by site-specific recombinase recognition sequences, and transiently expressing the site-specific recombinase.
(2) A transformed plant, from which the DNA flanked by site-specific recombinase recognition sequences has been removed according to the method of (1).
(3) A method for transposing a transposon that does not have a transposase, wherein the method comprises the steps of: using *Agrobacterium* to introduce a transposase-encoding DNA into a transformed plant that comprises the transposon having no transposase, and transiently expressing the transposase.
(4) A transformed plant, from which the transposon that does not have a transposase has been transposed according to the method of (3).
(5) A transformed plant which is an offspring or a clone of the transformed plant of (2) or (4).
(6) A reproductive material from the transformed plant of (2), (4), or (5).

The present inventors provide methods for removing a DNA flanked by site-specific recombinase recognition sequences, wherein the methods comprise the steps of: introducing a site-specific recombinase-encoding DNA into a transformed plant via an *Agrobacterium,* wherein the transformed plant comprises the DNA flanked by site-specific recombinase recognition sequences; and transiently expressing the site-specific recombinase. These methods can remove the DNA flanked by site-specific recombinase recognition sequences more efficiently than conventional methods. In addition, these methods are much simpler as compared to conventional methods because tissue culturing is not required. Moreover, it is thought that these methods can be applied to desired plants and have a broad range of applications.

In the present invention, a "site-specific recombinase" refers to an enzyme that catalyzes a site-specific recombination process, namely, a recombination process occurring at specific sites within or between DNA molecules. A "site-specific recombinase recognition sequence" refers to a sequence of approximately 40 base pairs and which is a recognition sequence for a site-specific recombinase. Site-specific recombinases possess the function of excising and circularizing a DNA fragment flanked by recognition sequences; they also carry out the reverse reaction (insertion of a circular molecule through recognition sequences).

Site-specific recombinases of the present invention are not particularly limited, and include examples such as the budding yeast-derived FLP recombinase which recognizes FRT sequences, *Zygosaccharomyces rouxii-derived* enzyme R which recognizes the RS sequences in the R/RS system (Onouchi H. *et al*. (1995) Mol. Gen. Genet. 247:653-660; Onouchi H. *et al*. (1991) Nucl. Acids Res. 19:6373-6378), bacteriophage P1-derived enzyme Cre which recognizes the lox sequences in the Cre/lox system (Albert H. *et al*. (1995) Plant J. 7:649-659; Liu Q. *et al*. (1998) Current Biol. 8:1300-1309; Abmemski K. *et al*. (1983) Cell 32:1301-1311).

In the present invention, DNAs flanked by site-specific recombinase recognition sequences are preferably marker genes, but are not limited thereto. The marker genes are not particularly limited and include, for example, drug resistance genes such as kanamycin resistance genes, hygromycin resistance genes, and bialaphos resistance genes, and fluorescence genes such as the GUS genes and GFP genes.

Furthermore, *Agrobacterium* of the present invention includes, for example, *Agrobacterium tumefaciens* EHA105, but is not limited thereto.

In the present invention, "a transformed plant comprising a DNA flanked by site-specific recombinase recognition sequences" can be produced by, for example, introducing the DNA flanked by site-specific recombinase recognition sequences into a plant cell, and reproducing plants from the plant cell.

In the present invention, vectors used for the transformation of plant cells are not limited as long as they can express the inserted DNA in the plant cells. Vectors that can be used include, for example, vectors comprising promoters (e.g., cauliflower mosaic virus 35S promoter) for constitutive gene expression in plant cells, and vectors comprising promoters that are inducibly activated by external stimuli. The term "plant cell" as used herein includes various forms of plant cells, such as culture cell suspensions, protoplasts, leaf sections, and calluses.

A vector can be introduced into plant cells by known methods, such as by using polyethylene glycol, electroporation, Agrobacterium-mediated transfer, and particle bombardment. Particle bombardment can be carried out by, for example, using equipment available from Bio-Rad. Plants can be regenerated from transformed plant cells by known methods and according to the type of the plant cell (see Toki S. *et al*. (1992) Plant Physiol. 100:1503).
For example, methods for producing a transformed rice plant include: (1) introducing genes into protoplasts using polyethylene glycol, then regenerating the plant (suitable for indica rice cultivars) (Datta S.K. (1995) in "Gene Transfer To Plants", Potrykus I. and Spangenberg Eds., pp. 66-74); (2) introducing genes into protoplasts using electric pulses, then regenerating the plant (suitable for japonica rice cultivars) (Toki S. *et al*. (1992) Plant Physiol. 100:1503); (3) introducing genes directly into cells by particle bombardment, then regenerating the plant (Christou *et al*. (1991) Biotechnology, 9:957); (4) introducing genes using *Agrobacterium,* then regenerating the plant (for example, "Extremely Rapid Transformation of Monocotyledon" [Patent No. 3141084]); and so on. These methods are already established in the art and are widely used in the technical field of the present invention. Such methods can be suitably used in the present invention.

Furthermore, "a transformed plant comprising a DNA flanked by site-specific recombinase recognition sequences" of the present invention can be produced by, for example, directly introducing a DNA flanked by site-specific recombinase recognition sequences into an intact plant, according to the method described below.

In the present invention, a DNA encoding a site-specific recombinase is introduced into the thus-produced transformed plant, which comprises the DNA flanked by site-specific recombinase recognition sequences, using *Agrobacterium.* As a result, the site-specific recombinase is transiently expressed, and the DNA flanked by the site-specific recombinase recognition sequences is removed.

In the present invention, in planta transformation methods can be suitably used to introduce a DNA encoding a site-specific recombinase into a transformed plant that comprises a DNA flanked by site-specific recombinase recognition sequences, via *Agrobacterium.*

Herein, the "in planta transformation methods" refer to methods of directly introducing genes into an intact plant, without performing callus induction or protoplast isolation from the plant. In particular, the methods developed for *Arabidopsis* are very simple methods, in which transformation is carried out by simply soaking the inflorescence of a plant at the budding stage in a suspension of *Agrobacterium* (in the vacuum infiltration method, the plant is treated under reduced-pressure after immersion). Transformation (introduction of a gene) is presumed to occur in egg cells, resulting in 0.1 to 0.5% of self-reproducing seeds becoming transformed.

Since these methods involve transformation without tissue culture, they have the advantages of: (1) low possibility of inducing somaclonal variation, (2) extremely simple operation, and (3) short time to obtain transformed plants, when compared with conventional methods.

Examples of the in planta transformation methods (Bent A.W. "Arabidopsis in Planta Transformation. Uses, Mechanisms, and Prospects for Transformation of Other Species." (2000) Plant Physiology 124:1540-1547) of the present invention, include, but are not limited to, for example, the floral dip method, the vacuum infiltration method, the incision/inoculation method, and the floral spray method which infects by spraying a suspension *of Agrobacterium* (Chung M.H. *et al*. (2000) Transgenic Res. 9(6):471-476).

In the present invention, when the DNA flanked by site-specific recombinase recognition sequences is a drug resistance gene or a fluorescence gene, the loss of drug resistance or fluorescence can be used to determine whether the DNA flanked by site-specific recombinase recognition sequences is removed from the transformed plant, into which the DNA encoding a site-specific recombinase has been introduced, or from the cells or seeds thereof. The Southern method, the PCR method, or such, can also be used.

Furthermore, the process of how a transposase recognizes specific sequences and excises a transposon is similar to the process of how a site-specific recombinase excises its recognition sequences. The present inventors provide methods for transposing a transposon that does not have a transposase, which comprise the steps of introducing a transposase-encoding DNA into a transformed plant that comprises the transposon having no transposase via *Agrobacterium,* and transiently expressing the transposase.

At present, gene tagging with transposons is mainly performed in a two-component system. In this method, a plant which comprises a transposon having no transposase (a non-autonomous transposon) is crossed with a plant that expresses a transposase to transpose the non-autonomous transposon. Then, to avoid the necessity of re-transferring, the transposase gene is segregated in the next generation. Thus, this method requires a tremendous amount of labor and time. In contrast, the methods of the present invention use *Agrobacterium* to introduce a transposase-encoding DNA into a plant comprising a non-autonomous transposon for transiently expressing the transposase and transferring the non-autonomous transposon. Segregation of the transposase gene in the following generation is not required, and high efficiency transposition is expected.

Transposases of the present invention are not particularly limited, and include, for example, the maize Ac/Ds system.

In the present invention, "a transformed plant which comprises a transposon having no transposase" can be produced by, for example, introducing a transposon that does not have a transposase into a plant cell, and reproducing a plant from the plant cell, or by introducing a transposon that does not have a transposase into a plant by an in planta transformation method. The present invention uses *Agrobacterium* to introduce a transposase-encoding DNA into the thus-produced transformed plant, which comprises the transposon having no transposase. This causes transient expression of the transposase, and results in transposition of the transposon which does not have a transposase.

Once a transformed plant is obtained, whether by removing a DNA flanked by site-specific recombinase recognition sequences from a transformed plant comprising the DNA, or by transferring a transposon that does not have a transposase from a transformed plant comprising the transposon having no transposase, the offspring can be obtained from these plants through sexual or vegetative propagation. Furthermore, it is also possible to obtain reproductive materials (for example, seeds, fruits, ears, tubers, root tubers, tubs, callus, and protoplasts, etc.) from these plants, or offspring or clones thereof, and to mass-produce these plants using them.

### Brief Description of the Drawings

Fig. 1 is a diagram of vectors used in the present invention: (A) pKO101, and (B) pSkp-1-FLP (for transient expression of FLP in *Arabidopsis).*
Fig. 2 is a diagram showing FLP excision of the region between FRT sequences. When the region between FRT sequences is excised upon the transient expression of FLP, the *mas* promoter and the *bar* gene which remain in the genome are joined, and the plant becomes bialaphos resistant. At the same time, the plant loses its hygromycin resistance because the excised DNA fragment cannot replicate in the cell and is disintegrated.

### Best Mode for Carrying out the Invention

Herein below, the present invention will be described in detail with reference to Examples; however, it is not to be construed as being limited thereto.

### [1] Construction of vectors for transient expression of the pKO101 and FLP genes

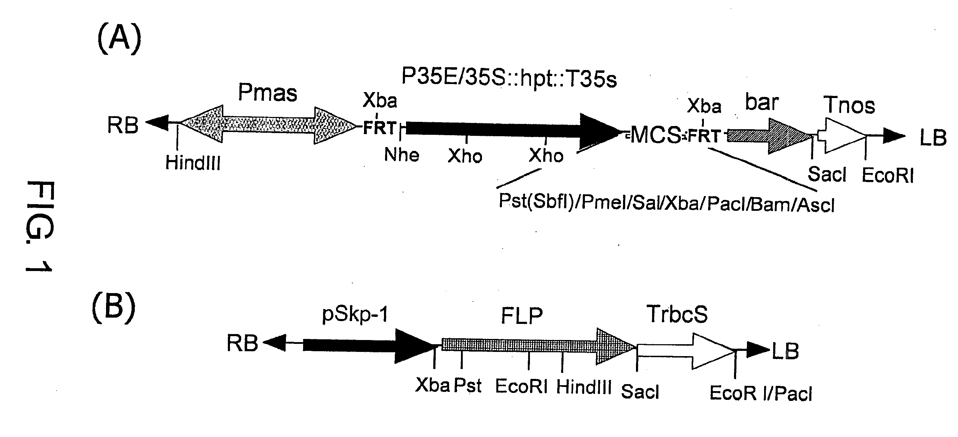

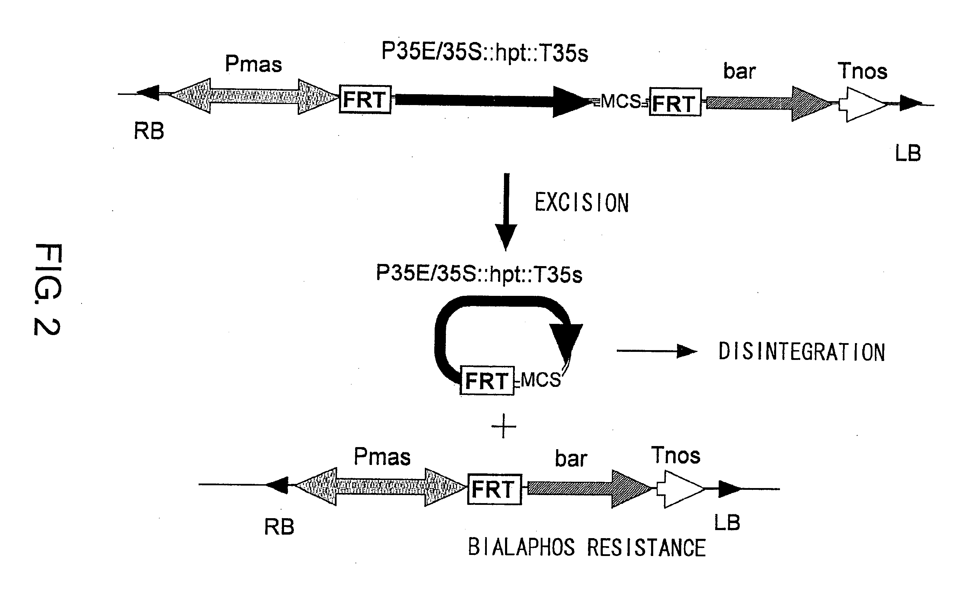

A construct was generated using a budding yeast-derived FLP recombinase (pOG44, Invitrogen) as the site-specific recombinase, wherein FRT sequences, which are FLP recombinase recognition sequences, were placed on both sides outside of the hygromycin-resistant gene cassette (CaMV35s promoter + hygromycin-resistant gene + CaMV35s terminator) (Fig. 1A). In this construct, the mannopine synthase gene promoter and the bialaphos resistance gene were placed outside of the FRT sequences. Expression of the bialaphos resistance gene was expected as a result of the FLP action, which results in the loss of the hygromycin resistance gene cassette flanked by FRT sequences (Fig. 2). Furthermore, the binary vector, pSkp-1FLP, shown in Fig. 1 was constructed to express the FLP gene (Fig. 1B). The above vectors were introduced into *Agrobacterium tumefaciens* EHA105 by the electroporation method.

### [2] Arabidopsis Transformation

*A. tumefaciens* EHA105 harboring pKO101 was used to transform the *Arabidopsis thaliana ecotype* Col-0 by the floral dip method (Clough, S.J. and Bent, A.F. (1998). Plant J. 16:735-743.). The obtained transformed seeds were seeded on the MS medium (Murashige, T. and Skoog, F. (1962) Physiol. Plant. 15:473-497.), which contains 20 mg/L of hygromycin and 0.4% Gel-Rite as a solidification agent. Plants which grew in the hygromycin-containing medium were used as candidates for the transformed plants in the experiment.

### [3] Removal of the marker gene through transient expression of the FLP gene

For transient expression of the FLP gene, the floral dip method was used to infect the transformed *Arabidopsis* plant obtained in [2], to which pKO101 had been introduced, with the pSkp-1FLP-retaining *A. tumefaciens* EHA105. When the region between FRT sequences is excised through the transient expression of the FLP gene, the *mas* promoter and the *bar* gene which had remained in the genome were joined, and the plant became bialaphos resistant. Accordingly, when bialaphos-resistant plants are efficiently obtained through transient expression of the FLP gene, the hygromycin resistance gene is judged to have been efficiently removed.

### Industrial Applicability

The present invention enables the efficient and easy excision and removal of DNA flanked by site-specific recombinase recognition sequences from a transformed plant comprising the DNA. It also enables the efficient and easy transposition of a transposon that does not comprise a transposase from a transformed plant which comprises the transposon having no transposase. It is thought that these methods can be applied to desired plants, and have an extremely broad range of applications.

## Claims

1. A method for removing a DNA flanked by site-specific recombinase recognition sequences, wherein the method comprises the steps of: using *Agrobacterium* to introduce a DNA encoding a site-specific recombinase into a transformed plant that comprises the DNA flanked by site-specific recombinase recognition sequences, and transiently expressing the site-specific recombinase.

2. A transformed plant, from which the DNA flanked by site-specific recombinase recognition sequences has been removed according to the method of claim 1.

3. A method for transposing a transposon that does not have a transposase, wherein the method comprises the steps of: using *Agrobacterium* to introduce a transposase-encoding DNA into a transformed plant that comprises the transposon having no transposase, and transiently expressing the transposase.

4. A transformed plant, from which the transposon that does not have a transposase has been transposed according to the method of claim 3.

5. A transformed plant which is an offspring or a clone of the transformed plant of claim 2 or 4.

6. A reproductive material from the transformed plant of claim 2, 4, or 5.
